# EUROPEAN PATENT APPLICATION

(11) **EP 1 710 305 A1**
(43) Date of publication of application: **11.10.2006**
(21) Application number: 04808045.1
(22) Date of filing: 22.12.2004
(51) Int. Cl.: C12N 15/00, C07K 16/18, C07K 16/42, G01N 33/53, G01N 33/543, C12P 21/08

(54) **FRACTION AND ANTIBODY COMPRISING GUINEA PIG IMMUNOGLOBULIN E**

(30) Priority: 26.12.2003 JP 2003434618; 01.10.2004 JP 2004289939
(71) Applicant: Dainippon Sumitomo Pharma Co., Ltd., Osaka 541-8524 (JP)
(72) Inventor: ITO, Koji, Chiba 2760046 (JP); HAYAKAWA, Shinobu, Kasugai-shi, Aichi 486-0923 (JP); FUNAOKA, Hiroyuki, c/o Dainippon Sumitomo Pharma, Suita-shi, Osaka 564-0053 (JP); HOSOE, Hiroaki, c/o Dainippon Sumitomo Pharma, Suita-shi, Osaka 564-0053 (JP); NISHIMURA, Makoto, c/o Dainippon Sumitomo Pharma, Suita-shi, Osaka 564-0053 (JP); OHKARU, Yasuhiko, c/o Dainippon Sumitomo Pharma, Suita-shi, Osaka 564-0053 (JP); YAKUO, Ikuhisa, c/o Dainippon Sumitomo Pharma, Suita-shi, Osaka 564-0053 (JP)
(74) Representative: Duckworth, Timothy John
(86) International application number: PCT/JP2004/019694
(87) International publication number: WO 2005/063978

(57) **Abstract**

Provided are a guinea pig immunoglobulin E fraction separated and purified from blood of an allergen-sensitized guinea pig, a highly specific anti-guinea-pig immunoglobulin E antibody obtained using the fraction as the antigen, and a reagent for immunoassaying guinea pig immunoglobulin E comprising the antibody.

## Description

### Technical Field

The present invention relates to a fraction comprising an essentially purified guinea pig immunoglobulin E (hereinafter also referred to as "IgE") having particular properties. The present invention also relates to an antibody capable of specifically recognizing guinea pig IgE (hereinafter also referred to as "anti-guinea-pig IgE antibody") produced by immunizing a non-human animal with the aforementioned fraction, and a reagent for immunoassaying guinea pig IgE, comprising the antibody.

### Background Art

If foreign matter enters a living body, an immune reaction occurs to eliminate the foreign matter. As a result of this immune reaction occurring in excess, various symptoms develop in the body; this condition is called allergy. Currently, patients with various allergic diseases, represented by atopic dermatitis and asthma, are increasing dramatically mainly among infants and young children, posing a social problem. Many of these allergic diseases are type I (immediate) allergies, which develop as IgE (antibody) against each allergen (antigen) is produced in excess.

Elucidation of the regulatory mechanism of the IgE production in type I allergies, and research and development of drugs that suppress allergies are ongoing. In the experimental settings for type I allergies as such, which are significantly mediated by IgE, guinea pigs are often used as experimental animals, as represented by, for example, passive cutaneous anaphylaxis (hereinafter referred to as "PCA reaction").

Japanese Patent Unexamined Publication No. 2000-266747 describes an anti-rat IgE antibody and a rat IgE assay kit comprising the antibody; Japanese Patent Unexamined Publication No. 2000-266748 describes an anti-mouse IgE antibody and a mouse IgE assay kit comprising the antibody. These two references do not describe an anti-guinea-pig IgE antibody or a guinea pig IgE antibody assay kit (reagent) comprising the antibody.

"Int. Archs Allergy Appl. Immun.", 1985, Vol. 77, pp. 438-444 describes an anti-guinea-pig IgE antibody produced by separating an IgE-rich fraction from serum of a parasite-infected guinea pig, and immunizing a rabbit with the fraction as the antigen. However, the fraction rich in guinea pig IgE described therein is considered to also contain considerable amounts of other classes of immunoglobulins, for example, immunoglobulin G. For this reason, the antibody (antiserum) obtained using the fraction as the antigen is poorly specific for guinea pig IgE; to obtain an anti-guinea-pig IgE antibody therefrom, the antiserum has needed to be neutralized with normal guinea pig serum. "Int Arch Allergy Appl Immunol", 1988, Vol. 87, pp. 424-429 and "ANNALS OF ALLERGY", 1981, Vol. 47, pp. 52-56 describe fractions and antibodies similar to those described in the aforementioned references.

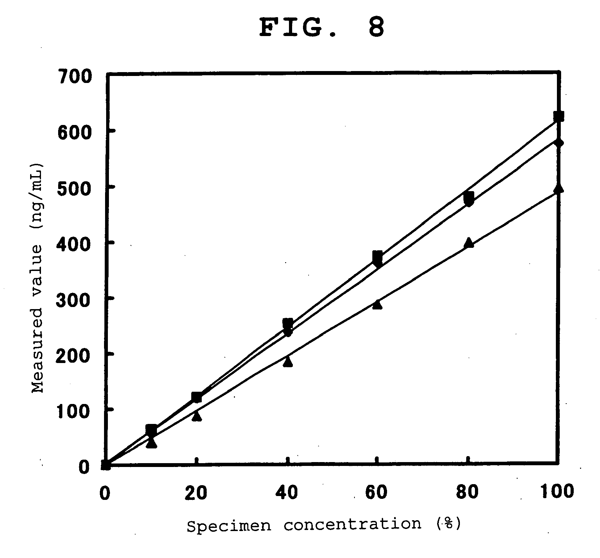

The "Journal of Immunological Methods", 1991, Vol. 139, pp. 123-134 discloses a method of separating each class of immunoglobulin from an immunoglobulin fraction in guinea pig blood. In Figure 8 of the reference, the results of SDS-PAGE (sodium dodecyl sulfate-polyacrylamide gel electrophoresis) of individual fractions separated by chromatography are shown, but the results for fractions 3 and 4, which reportedly contain IgE with PCA reaction activity as the index, do not include a unique major band distinguishable from other fractions. It is considered that this is because only a very small amount of IgE, which cannot be detected by SDS-PAGE, is present in the aforementioned two fractions.

Even if such a fraction is used as the antigen, a highly specific anti-guinea-pig IgE antibody cannot be produced.

As described above, an excellently specific anti-guinea-pig IgE antibody and a highly pure antigen for producing the antibody, that is, guinea pig IgE of high purity, have not been produced to date, nor has been known the properties thereof.

### Disclosure of the Invention

As described above, it is current practice to perform the guinea pig PCA reaction as an experimental system for allergies mediated by IgE. However, the PCA reaction does not quantitatively measure IgE itself. In the PCA reaction, no more than an estimation of the intensity of allergic reaction is performed by stimulating IgE-bound mast cells with an antigen to cause chemical mediators (histamine, leukotriene and the like) to be released and to act on blood vessels, and measuring the amount of pigment leaking therefrom.

In this situation, there has been a demand for the development of a highly specific anti-guinea-pig IgE antibody and a reagent for highly accurate guinea pig IgE assay comprising the antibody, in the experimental settings for allergies.

It is an object of the present invention to provide a fraction comprising highly purified guinea pig IgE, an excellently specific anti-guinea-pig IgE antibody produced using the fraction as the antigen, and a reagent for accurate and convenient immunoassay reagent for guinea pig IgE having high measurement sensitivity.

The present inventors have purified a fraction comprising IgE from an immunoglobulin fraction separated from blood of a guinea pig sensitized with an allergen, particularly with ovalbumin (hereinafter also referred to as "OVA"), and identified the properties thereof. Hence, the inventors have found that an anti-guinea-pig IgE antibody produced using this fraction as the antigen specifically recognizes guinea pig IgE, and that guinea pig IgE can be measured accurately at high sensitivity using an IgE immunoassay system composed of this antibody, and have developed the present invention.

Accordingly, the present invention provides the following:
[1] A fraction comprising essentially purified guinea pig immunoglobulin E having a purity sufficient to obtain a single major band of immunoglobulin E by SDS-PAGE, which is separated from the blood of an allergen-sensitized guinea pig, and having the following properties;
   (1) having a molecular weight of said major band of about 200 kDa,
   (2) being usable as an antigen for producing an antibody capable of specifically recognizing guinea pig immunoglobulin E,
   (3) being positive in a passive cutaneous anaphylaxis reaction in guinea pig,
   (4) being substantially free of guinea pig immunoglobulin G.
[2] The fraction of [1] above, wherein the guinea pig immunoglobulin E has a molecular weight of the major band of 187 to 195 kDa.
[3] The fraction of [1] or [2] above, wherein the guinea pig immunoglobulin E has a molecular weight of about 186 kDa as determined by mass spectrometry.
[4] The fraction of [1] to [3] above, wherein the aforementioned major band is separated into two bands by reducing SDS-PAGE, and the molecular weights of the individual bands after the separation are about 70 kDa and about 30 kDa.
[5] The fraction of [1] to [4] above, wherein the allergen is ovalbumin.
[6] An antibody capable of specifically recognizing guinea pig immunoglobulin E, produced by immunizing a non-human animal with the fraction comprising immunoglobulin E of [1] to [5] above.
[7] The antibody of [6] above, wherein the antibody is a monoclonal antibody.
[8] The antibody of [6] or [7] above, wherein the antibody exhibits substantially no cross-reaction with guinea pig immunoglobulin G or immunoglobulin M.
[9] An antibody capable of specifically recognizing guinea pig immunoglobulin E wherein the cross-reaction rate of the antibody with guinea pig immunoglobulin G or immunoglobulin M is less than 0.0001%.
[10] A reagent for immunoassaying guinea pig immunoglobulin E comprising the antibody of [6] to [9] above.
[11] A reagent for immunoassaying guinea pig immunoglobulin E comprising the antibody of [6] to [9] above and an enzyme-labeled antibody thereof.
[12] The reagent of [10] or [11] above, wherein the reagent for immunoassay is a reagent for performing sandwich enzyme-linked immunosorbent assay.
[13] A reagent for immunoassaying guinea pig immunoglobulin E comprising the immobilized antibody of [6] to [9] above and ovalbumin labeled with a labeling substance.
[14] A reagent for immunoassaying guinea pig immunoglobulin E comprising immobilized ovalbumin and the antibody of [6] to [9] above as labeled with a labeling substance.
[15] The reagent of [13] or [14], wherein the labeling substance is an enzyme.
[16] The reagent of any of [13] to [15] above, wherein the guinea pig immunoglobulin E is ovalbumin-recognizing immunoglobulin E.
[17] The antibody of [6] to [9] above, which is immobilized on a solid phase.
[18] The antibody of [17] above, which is used for producing or purifying guinea pig immunoglobulin E.

According to the present invention [1] above, a highly purified antigen for producing an anti-guinea-pig IgE antibody can be provided. The present invention [6] above provides an excellently specific anti-guinea-pig IgE antibody, and it is possible to accurately and conveniently measure guinea pig IgE in a biological sample such as blood at high sensitivity using the present invention [10] above comprising the antibody.

According to the present invention [13], guinea pig immunoglobulin E that recognizes OVA by an antigen-antibody reaction can be specifically measured.

According to the present invention [17], guinea pig immunoglobulin E can be produced or purified to high purity.

### Brief Description of the Drawings

Figure 1 shows an analysis by SDS-PAGE showing the molecular weight of the major band of the guinea pig IgE fraction of the present invention.
Figure 2 shows an analysis of the guinea pig IgE fraction of the present invention by reducing SDS-PAGE.
Figure 3 shows the results of an experiment of the guinea pig PCA reaction of the guinea pig IgE fraction of the present invention.
Figure 4 shows an analysis by Western blotting indicating that the guinea pig IgE fraction of the present invention is substantially free from IgG.
Figure 5 shows a standard curve generated using a reagent for guinea pig IgE immunoassay of the present invention.
Figure 6 shows the results of a dilution test of a reagent for guinea pig IgE immunoassay of the present invention (specimens 1 to 3).
Figure 7 shows the standard curve generated in term (3) of Example 5.
Figure 8 shows the results of the dilution test in term (4)-(a) of Example 5.
Figure 9 shows the results of the correlation test for the PCA reaction in term (4)-(e) of Example 5.
Figure 10 shows an analysis by SDS-PAGE of the guinea pig IgE fraction of the present invention used to generate a molecular weight curve.
Figure 11 shows a mass analysis of the guinea pig IgE fraction of the present invention.

### Best Mode for Embodying the Invention

The present invention relates to a fraction separated from blood of an allergen-sensitized guinea pig, and comprising guinea pig immunoglobulin E having particular properties (hereinafter referred to as "guinea pig IgE fraction").

The guinea pig IgE fraction of the present invention has been purified from an immunoglobulin fraction of blood of an allergen-sensitized guinea pig.

The allergen is not subject to limitation, as long as it allows IgE to be produced in excess in the blood of a guinea pig when sensitized therewith, and any one known in the art can be used. As examples of such allergens, OVA, mite antigen, parasite, parasite-extracted protein, equine serum and the like can be mentioned; of these, OVA is preferred because of the ease of obtainment and handling.

Sensitization can be performed by a conventional method, for example, by intraperitoneally administering an allergen to a guinea pig over a given period. An immunoglobulin fraction can be produced by collecting blood from a guinea pig thus sensitized with an allergen, and performing a commonly known treatment such as saturated ammonium sulfate salting-out.

The guinea pig IgE fraction of the present invention can be produced from thus obtained guinea pig immunoglobulin fraction by combining a plurality of physical or chemical means known per se.

For example, to remove immunoglobulin G, which is contained in large amounts in the immunoglobulin fraction, a method employing a Protein G column or DE52 anion exchange resin is used. For the purification of a guinea pig IgE fraction, anion exchange treatments such as Q-Sepharose™ column chromatography and MonoQ™ column chromatography, gel filtration column treatments such as Superdex™ 200 column chromatography, and the like are performed. Each of these physical or chemical means may be repeatedly performed as necessary.

In the above-described individual physical and chemical means, a solvent usually used according to each means can be used as appropriate without limitation.

More specifically, the guinea pig IgE fraction of the present invention can be produced by the method described in an Example below.

The guinea pig IgE fraction of the present invention thus obtained has the various properties described above, as shown in an Example below.

It is preferable that the purity of the IgE contained in the guinea pig IgE fraction of the present invention be as high as possible because a more highly specific anti-IgE antibody can be obtained when the fraction is used as the antigen.

The present invention also relates to an antibody capable of specifically recognizing guinea pig immunoglobulin E, produced by immunizing a non-human animal with the above-described guinea pig IgE fraction.

While the antibody of the present invention may be any of a polyclonal antibody and a monoclonal antibody, as long as it is an antibody that specifically recognizes guinea pig IgE, a monoclonal antibody is preferred because of higher specificity and uniformity.

A polyclonal antibody can be produced by immunizing a non-human animal using the guinea pig IgE fraction of the present invention as the antigen. Concretely, a polyclonal antibody can be produced by immunizing a non-human animal such as a rabbit, mouse, rat, sheep, goat, pig, or chicken with a mixture of a guinea pig IgE fraction obtained as described above and an appropriate adjuvant, collecting blood from the animal, and subjecting the blood to a commonly known treatment. A monoclonal antibody can be produced by collecting splenocytes from an animal thus immunized, performing cell fusion with myeloma cells by the method of Milstein et al., screening of antibody-producing cells, cloning and the like to establish a cell line that produces the anti-guinea-pig IgE antibody, and culturing this cell line.

The anti-guinea-pig IgE antibody thus obtained is very highly specific for guinea pig IgE, and does not recognize other classes of immunoglobulins, for example, immunoglobulin G, which is reportedly normally present in much larger amounts than IgE in blood.

The anti-IgE antibody of the present invention can be suitably used in the reagent for immunoassay of the present invention described below.

Furthermore, the present invention relates to a reagent for guinea pig IgE immunoassay comprising the above-described anti-guinea-pig IgE antibody.

The reagent for guinea pig IgE immunoassay of the present invention is based on the high specificity of the anti-guinea-pig IgE antibody of the present invention for the antigen, and is useful as a reagent for performing various immunoassays. The immunoassay as used herein may be any assay comprising an antigen-antibody reaction; as examples, enzyme immunoassay (EIA method), latex aggregation, immunochromatography, radioimmunoassay (RIA method), fluorescence immunoassay (FIA method), luminescence immunoassay, evanescent wave analysis and the like can be mentioned. Of these, the reagent of the present invention for performing the EIA method or latex aggregation is preferred from the viewpoint of the ease of operation.

When a reagent for performing the EIA method has been chosen as the reagent for immunoassay of the present invention, the EIA method is preferably sandwich enzyme-linked immunosorbent assay (sandwich ELISA method) using two kinds of anti-guinea-pig IgE antibodies that recognize distinct epitopes on guinea pig IgE.

As a kind of the sandwich ELISA method, a method employing the avidin-biotin reaction is also available. Regarding the assay principle, this method comprises capturing guinea pig IgE in the sample by means of an immobilized anti-guinea-pig IgE antibody, performing an antigen-antibody reaction between the captured guinea pig IgE and a biotin-labeled antibody, then adding enzyme-labeled streptavidin to cause the avidin-biotin reaction.

As such, the sandwich ELISA method is excellently specific for antigens because of the use of two kinds of antibodies, and is capable of accurately measuring guinea pig IgE in a sample wherein other classes of immunoglobulins are also present.

A reagent of the present invention for performing the sandwich ELISA method is comprised of an immobilized anti-guinea-pig IgE antibody and an enzyme- or biotin-labeled anti-guinea-pig IgE antibody.

The two kinds of anti-guinea-pig IgE antibodies contained in the reagent for performing the sandwich ELISA method may be of any combination of monoclonal antibodies, of polyclonal antibodies, or of a monoclonal antibody and a polyclonal antibody.

An immobilized anti-guinea-pig IgE antibody can be produced by binding an antibody obtained as described above to a solid phase, for example, microplate wells, plastic beads, magnetic beads, or a carrier for chromatography (e.g., Sepharose™). The binding to the solid phase can normally be achieved by dissolving the antibody in an appropriate buffer solution such as a citrate buffer solution, and bringing the solid phase surface and the antibody solution into contact with each other for an appropriate time (1 to 2 days). The immobilized anti-guinea-pig IgE antibody can be used not only for measuring guinea pig IgE, but also for producing or purifying guinea pig IgE on the basis of the property thereof of offering a good recovery rate for guinea pig IgE, as shown in the addition-recovery test in Example 4 below.

Furthermore, to suppress nonspecific adsorption or nonspecific reaction, it is common practice to bring a phosphate-buffered solution of bovine serum albumin (BSA), bovine milk protein and the like into contact with the solid phase, so as to block a surface portion of the solid phase, which is not coated by the antibody, with the aforementioned BSA, bovine milk protein and the like.

An enzyme-labeled anti-guinea-pig IgE antibody can be produced by linking (labeling) an anti-guinea-pig IgE antibody that recognizes an epitope different from the above-described immobilized antibody and an enzyme. As the enzyme that labels the antibody, peroxidase, alkaline phosphatase, glucose oxidase, β-galactosidase and the like can be mentioned. The linking of these enzymes and the anti-guinea-pig IgE antibody can be performed by a method known per se, for example, the glutaraldehyde method, maleimide method and the like.

A biotin-labeled anti-guinea-pig IgE antibody can be produced by linking biotin and an anti-guinea-pig IgE antibody by a method obvious to those skilled in the art. For example, it is possible to link biotin and an anti-guinea-pig IgE antibody using a commercially available biotin labeling kit.

In addition to the above-described anti-guinea-pig IgE antibody, the reagent of the present invention for performing the sandwich ELISA method may comprise a standard substance, a washing solution, a reagent for assaying an enzyme activity (substrate agent, substrate-lysing solution, quenching solution and the like) and the like as constituent reagents as necessary. The reagent of the present invention for performing the method employing the avidin-biotin reaction may further comprise enzyme-labeled streptavidin.

A substrate agent to be contained in the reagent as desired is appropriately selected according to the labeling enzyme employed. For example, when peroxidase is chosen as the enzyme, o-phenylenediamine (OPD), tetramethylbenzidine (TMB) and the like are used; when alkaline phosphatase has been chosen, p-nitrophenylphosphate (PNPP) and the like are used. For the quenching solution and substrate-lysing solution, traditionally known ones can be used as appropriate without limitation according to the selected enzyme.

A reagent for performing latex aggregation, which is another preferred embodiment of the present invention, comprise the anti-guinea-pig IgE antibody in the form of a latex-sensitized anti-guinea-pig IgE antibody. Sensitization (linking) of latex particles and antibody can be achieved by a method known in the art, for example, chemical binding employing carbodiimide, glutaraldehyde and the like as the crosslinking agent or physical adsorption.

A reagent for performing latex aggregation may comprise a buffer solution for dilution stability, a standard substance and the like as necessary, in addition to the above-described latex-sensitized anti-guinea-pig immunoglobulin antibody.

Reagents for performing radioimmunoassay (RIA method), fluorescence immunoassay (FIA method), luminescence immunoassay and the like, which are other embodiments of the present invention, can also be produced according to a conventional method as with the reagents for performing the above-described EIA method and latex aggregation.

A reagent for guinea pig IgE immunoassay of the present invention thus produced is capable of specifically measuring immunoglobulin E only, and does not cross-react with other classes of immunoglobulins, for example, immunoglobulin G and immunoglobulin M, as shown in an Example below.

The present invention also relates to a reagent for guinea pig immunoglobulin E immunoassay comprising the aforementioned immobilized anti-guinea-pig IgE antibody and OVA labeled with a labeling substance.

The aforementioned immobilized anti-guinea-pig IgE antibody can be produced in the same manner as with the above-described sandwich ELISA.

As the labeling substance that labels OVA, an enzyme, a fluorescent substance, a radioactive substance and the like can be mentioned. As specific examples of the enzyme, peroxidase, alkaline phosphatase, glucose oxidase, β-galactosidase and the like can be mentioned. As examples of the fluorescent substance, fluorescein or derivatives thereof can be mentioned; as an example of the radioactive substance, ¹²⁵I can be mentioned. Of these labeling substances, enzymes are preferred because of the ease of handling.

Labeled OVA can be produced by linking OVA and a labeling substance by a commonly known method such as the glutaraldehyde method, periodate method or maleimide method. The specific method of producing, for example, enzyme-labeled OVA, is as shown in an Example below.

A reagent for immunoassay of the present invention thus produced, comprising an immobilized anti-guinea-pig IgE antibody and labeled OVA, is one to specifically measure the concentration of OVA-recognizing guinea pig IgE. The mechanism of the measurement is described below.

When IgE in the specimen is captured by the anti-guinea-pig IgE antibody of the present invention as immobilized on a microplate and the like, and OVA labeled with a labeling substance is added thereto, the labeled OVA binds only to OVA-recognizing IgE out of the captured IgE; by measuring the amount of the labeling substance by a commonly known method, OVA-recognizing guinea pig IgE can be specifically measured.

In the above-described mechanism, OVA-recognizing guinea pig IgE can also be measured by using immobilized OVA in place of the immobilized antibody, and a labeled antibody in place of labeled OVA. Accordingly, a reagent of the present invention may be embodied in the form of a reagent for guinea pig IgE immunoassay comprising immobilized OVA and the aforementioned anti-guinea-pig IgE antibody as labeled with a label.

Immobilization of OVA can be performed in the same manner as with the aforementioned anti-guinea-pig IgE antibody.

As such, a reagent for guinea pig IgE immunoassay may comprise a standard substance, a washing solution and the like as constituent reagents as necessary, as with the reagent based on the sandwich ELISA method described above. When the labeling substance is an enzyme, a reagent for assaying an enzyme activity such as a substrate agent, a substrate-lysing solution, and a quenching solution may further be contained as constituent reagents.

### Examples

The present invention is hereinafter described in more detail by means of the following Examples, which, however, are not to be construed as limiting the scope of the invention. Example 1: Production of guinea pig IgE fraction

### (1) Allergen sensitization

10 µg of OVA and 4 mL of aluminum hydroxide gel were mixed and intraperitoneally administered to guinea pigs (Hartley, about 600 g, female). Immediately thereafter, pertussis vaccine (2.5 x 10¹⁰ cells/individual) was intraperitoneally administered in the same manner (first sensitization). Twelve days later, cyclophosphamide was intraperitoneally administered at a dose of 250 mg/kg (physiological saline 4 mL). Subsequently, the animals were sensitized 8 times at 2-week intervals in the same manner as the first sensitization. After completion of the series of sensitization, each animal was thoracotomized under diethyl ether anesthesia, and blood was drawn from the heart. The blood was centrifuged (3000 rpm, 10 min, 4°C), and 35 mL of serum was collected.

### (2) Separation of immunoglobulin fraction

The serum obtained in (1) above was centrifuged (18,000 rpm x 15 min), and an equal volume of a 20 mM sodium phosphate buffer solution (pH 7.0) was added to the supernatant. To this mixed liquid, an equal volume of saturated ammonium sulfate solution was added drop by drop with stirring under ice cooling (50% saturated ammonium sulfate); after the addition, the liquid was stirred for 20 minutes. Next, the liquid was centrifuged (18,000 rpm x 30 min), the obtained precipitate was dissolved in a 10 mM sodium phosphate buffer solution (pH 7.0), and the solution was dialyzed against a 10 mM sodium phosphate buffer solution (pH 7.0) to obtain an immunoglobulin fraction liquid.

### (3) Purification of guinea pig IgE fraction

The immunoglobulin fraction liquid obtained in (2) above was centrifuged (18,000 rpm x 30 min) and the turbid material in the inner solution was removed, after which the solution was developed on a column, packed with 100 mL of anion exchange resin (DE52), and equilibrated with a 10 mM sodium phosphate buffer solution (pH 7.0). After the column was washed with a 10 mM sodium phosphate buffer solution (pH 7.0), IgE was eluted with a 35 mM sodium phosphate buffer solution (pH 7.0). The IgE-eluted fraction from DE52 was 50% saturated with ammonium sulfate, and stirred for 20 minutes, followed by centrifugation (18,000 rpm x 30 min). The precipitate obtained was dissolved in a 20 mM sodium phosphate buffer solution (pH 7.0), and this solution was dialyzed against the same buffer solution to obtain an IgE-containing solution. The IgE-containing solution after ammonium sulfate purification was developed on a Protein G column (Protein G Sepharose 4 Fast Flow [trade name], column size; 1.6 x 3 cm, Amersham Biosciences K.K.), equilibrated with a 20 mM sodium phosphate buffer solution (pH 7.0) (flow rate 0.5 mL/min). A flow through fraction with the same buffer solution was collected. This fraction was again developed on a Protein G column under the same conditions, and the resulting flow through fraction was collected and dialyzed against a 10 mM Na,K phosphate buffer solution, pH 7.5.

The inner dialytic liquid after Protein G treatment was developed on a Q-Sepharose F.F. column (Q-Sepharose Fast Flow [trade name], column size; 1.6 x 8.5 cm, Amersham Biosciences K.K.) (Buffer A = 10 mM Na,K phosphate buffer solution, pH 7.5, Buffer B = 0.3 M Na,K phosphate buffer solution, pH 7.5, flow = 3 mL/min, fraction size = 3 mL, gradient = 0% B for 5 min. 0-100% B in 90 min). The IgE-eluted fraction was determined by the PCA reaction test described below.

The Q-Sepharose IgE-eluted fraction liquid was concentrated by ultracentrifugation, and developed on a Superdex 200 column (Superdex 200XK16/60 [trade name], column size; 1.6 x 60 cm, Amersham Biosciences K.K.) (flow rate; 1 mL/min, fraction size; 1 mL, eluent; 50 mmol/L MES, 0.1 mol/L NaCl, pH 6.5). The IgE-eluted fraction was determined by PCA reaction test, and was dialyzed against a 10 mM Na,K phosphate buffer solution, pH 7.5.

The IgE-eluted fraction from the Superdex 200 column was developed on a Mono Q column (Mono Q HR5/5 [trade name], column size; 0.5 x 5 cm, Amersham Biosciences K.K.) (Starting buffer (buffer A): 0.01 M Na,K phosphate buffer pH 7.5, Gradient buffer (buffer B): 0.3 M Na,K phosphate buffer pH 7.5, Gradient: after sample charge, 0% B for 5 min, 0-100% B in 30 min, flow rate 1.0 ml/min, UV range 0-0.2). The IgE-eluted fraction was determined by the PCA reaction test described below, and a guinea pig IgE fraction was obtained.

### (4) Various properties of guinea pig IgE fraction

Various properties of the guinea pig IgE fraction obtained in (3) above were as follows:
[1] A single major band of IgE is obtained by SDS-PAGE, and the molecular weight of the major band is about 200 kDa. SDS-PAGE (1)

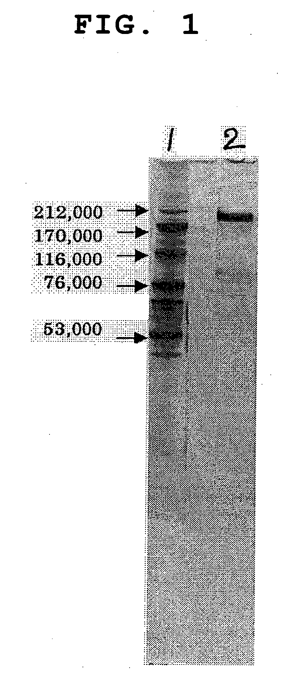

The guinea pig IgE fraction was dissolved in a buffer solution for non-reduced sample (62.5 mmol/L Tris-HCl, pH 6.8, 25% glycerol, 2% SDS and 0.01% bromophenol blue); after heating at 100°C for 3 minutes, SDS-PAGE was conducted. The sample was separated on a gel with an acrylamide concentration gradient from 5 to 20% (E-T520L model, ATTO Corporation). The electrophoretic buffer solution used was 62.5 mmol/L Tris-HCl, pH 8.3, 192 mmol/L glycine, 0.1% SDS. After electrophoresis at 40 mA for 80 minutes, the gel was stained with silver (Silver Staining Kit Wako, Wako Pure Chemical Industries, Ltd.). The molecular weight markers used were in the HMW SDS marker kit (myosin [212,000 Da], α₂ macroglobulin [170,000 Da], β-galactosidase [116,000 Da], transferrin [76,000 Da], glutamate dehydrogenase [53,000 Da], Amersham Biosciences K.K.). As shown in Figure 1, a single primary band was observed at a molecular weight of approximately 200 kDa (lane 1; marker, lane 2; guinea pig IgE fraction).

### SDS-PAGE (2)

To determine the accurate molecular weight, SDS-PAGE was performed in the same manner as described above using a gel having an acrylamide concentration of 7.5% (E-T7.5L model, ATTO Corporation). The gel was subjected to Coomassie staining (Bio-safe Coomassie, Bio-Rad Company). A molecular weight curve was generated using the same molecular weight markers as described above, and the molecular weight range of this fraction was determined. As a result, the molecular weight ranged from 187 kDa to 195 kDa.

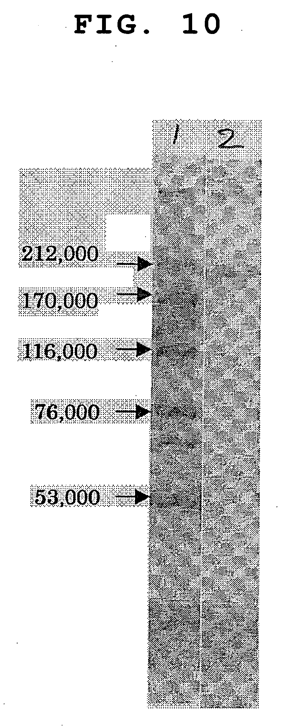

Figure 10 shows the results of SDS-PAGE at this time (lane 1; marker, lane 2; guinea pig IgE fraction). Mass spectrometry

A mass spectrometry of the guinea pig IgE fraction was performed under the conditions shown below.
Instrument: AXIMA-CFR plus (Shimadzu Corporation)
Laser light source: Nitrogen inclusion type laser (λ=337.1 nm) Detection ion: Cation
Flight mode: Linear mode
Acceleration voltage: 20 kV
Delayed extraction; ON (optimized to m/z180000)
Beam blanking: Ion detection not more than m/z10000 was excluded
Matrix solution: Prepared by dissolving 20 mg of sinapinic acid in a mixed solvent of 350 µL of acetonitrile and 350 µL of 0.1% TFA (trifluoroacetic acid).
Mass calibration: The 160 kDa Mass Calibrant in the Invitromass High Molecular Weight Mass Calibration Kit (Invitrogen Corporation) was used. Using the [M+H]⁺m/z156081, [M+2H]²⁺79541 and [3M+2H]²⁺238621 of the 160 kDa Mass Calibrant, calibration was performed by the external standard method.
Sample preparation: 0.5 µL of sample stock solution (guinea pig immunoglobulin fraction) was applied to a sample plate, immediately after which 0.5 µL of matrix solution was added; after the plate was air dried, a measurement was performed.

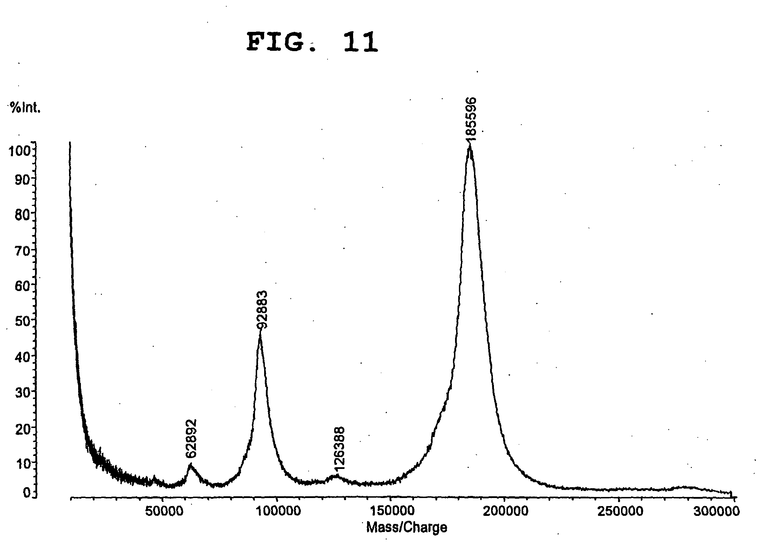

As a result of the measurement, m/z185596 was detected as the main component, as shown in Figure 11. Note that m/z92883 and m/z62892 are attributable to the [M+2H]²⁺ and [M+3H]³⁺ ions, respectively, of the main component, and m/z126388 is attributable to the [2M+3H]³⁺ ion of the main component. Hence, the measured value of the molecular weight of the guinea pig IgE contained in this sample was 185596. [2] The aforementioned major band is separated into two bands by reducing SDS-PAGE, and the molecular weights of the individual bands after the separation are about 70 kDa and about 30 kDa.

The guinea pig IgE fraction was dissolved in a buffer solution for reduced samples (62.5 mmol/L Tris-HCl, pH 6.8, 350 mmol/L dithiothreitol, 25% glycerol, 2% SDS and 0.01% bromophenol blue) and heated at 100°C for 3 minutes, after which SDS-PAGE was conducted. The sample was separated on a gel with an acrylamide concentration gradient from 5 to 20% (E-T520L model, ATTO Corporation). The electrophoretic buffer solution used was 62.5 mmol/L Tris-HCl, pH 8.3, 192 mmol/L glycine, 0.1% SDS. After electrophoresis at 40 mA for 80 minutes, the gel was stained with silver in the same manner as described above. The molecular weight markers used were in the LMW marker kit (phosphorylase b [97,000 Da], albumin [66,000 Da], ovalbumin [43,000 Da], carbonic anhydrase [30,000 Da], trypsin inhibitor [20,100 Da], α-lactalbumin [14,400 Da], Amersham Biosciences K.K.).

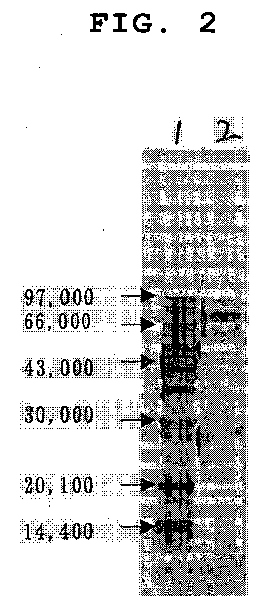

As shown in Figure 2, the about 200-kDa major band observed in [1] above was not observed by reducing SDS-PAGE of the guinea pig IgE fraction, whereas an about 70-kDa band and an about 30-kDa band were observed (lane 1; marker, lane 2; guinea pig IgE fraction).

### [3] Can be used as an antigen for producing an antibody capable of specifically recognizing guinea pig IgE.

This property is described in Example 2 below.

### [4] Positive for the PCA reaction in the guinea pig.

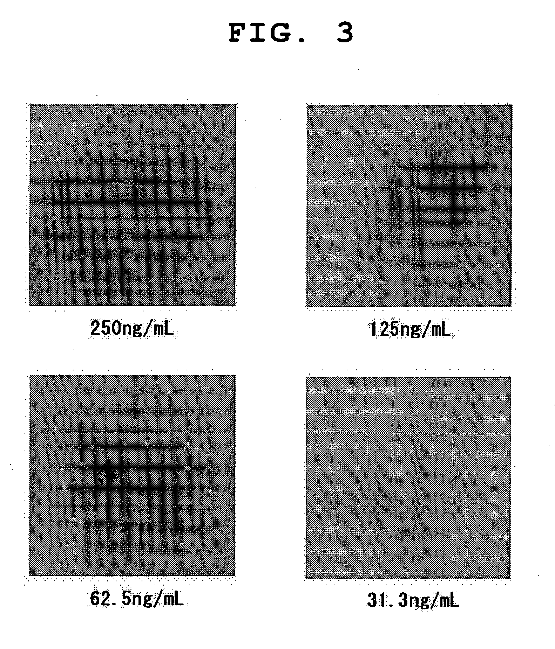

The guinea pig IgE fraction was diluted with physiological saline to prepare a series of dilutions having protein concentrations of 31.3, 62.5, 125 and 250 ng/mL. Guinea pigs (Hartley, female, 450 to 600 g) were anesthetized with ether and shaven in the back, and 0.1 mL of each serial preparation was intradermally injected. After the guinea pigs were reared for 8 days, 1 mL of a solution of 1 mg/mL OVA and 1% Evans Blue in physiological saline was injected from a front leg, and the animals were allowed to stand for 30 minutes. As shown in Figure 3, on the back of the skin after sacrificing, a blue spot was observed with dependence on the protein concentration of the guinea pig IgE fraction.

### [5] Substantially free from guinea pig immunoglobulin G.

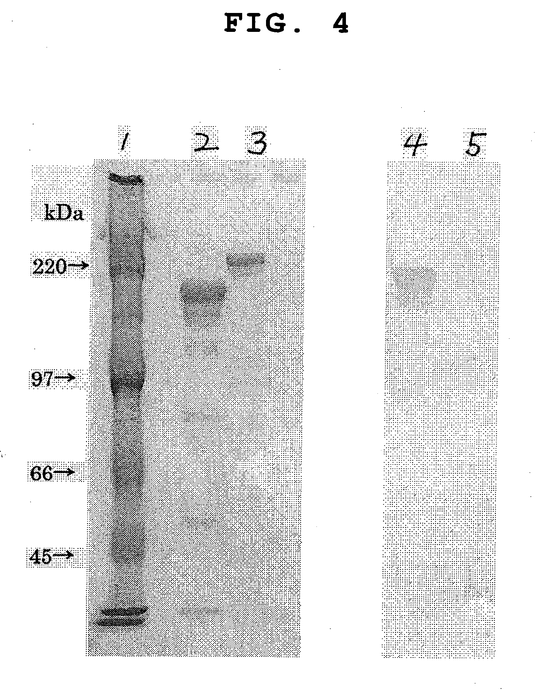

By Protein G column chromatography in the process for purifying the guinea pig IgE fraction, the column-adsorbed IgG was eluted to obtain a guinea pig IgG fraction. The guinea pig IgG fraction and the guinea pig IgE fraction were subjected to SDS-PAGE under the above-described non-reducing conditions (see Figure 4, lane 1; marker, lane 2; guinea pig IgG fraction, lane 3; guinea pig IgE fraction).

The separated protein in the gel was transferred to a nitrocellulose membrane using HorizBlot (ATTO Corporation) (at 131 mA for 1 hour). The nitrocellulose membrane after the transfer was shaken in a blocking solution (2% Block Ace [registered trademark] powder) at room temperature for 30 minutes, after which the membrane was shaken in a washing solution (20 mmol/L Tris, 500 mmol/L NaCl, 0.05% Tween [registered trademark]-20, pH 7.5) at room temperature for 10 minutes. A goat anti-guinea-pig IgG antibody (Chemicon International Inc.) was diluted with the blocking solution to obtain a final antibody concentration of 10 µg/mL. The nitrocellulose membrane after washing was shaken with the goat anti-guinea-pig IgG antibody, previously diluted to 10 µg/mL, at room temperature for 1 hour. The nitrocellulose membrane was shaken in the washing solution at room temperature for 5 minutes, and the washing solution was removed, after which the washing solution was added, and the membrane was again shaken at room temperature for 5 minutes. The nitrocellulose membrane after washing was shaken in a solution of horseradish-peroxidase-labeled swine anti-goat IgG antibody (Dainippon Pharmaceutical Co., Ltd.) diluted 2000 fold with the blocking solution at room temperature for 1 hour. Shaking in the washing solution at room temperature for 5 minutes was twice repeated, and the membrane was shaken in 20 mmol/L Tris, 500 mmol/L NaCl, pH 7.5 at room temperature for 5 minutes. The nitrocellulose membrane was shaken with the reagent Developer in the Immun-blot assay kit (Bio-Rad Laboratories, Inc.) at room temperature for 5 minutes to develop the color reaction, and was washed with distilled water to stop the reaction.

As shown in Figure 4, with the guinea pig IgG fraction sample, intense color development mediated by guinea pig IgG was observed near a molecular weight of approximately 160 kDa (lane 4; guinea pig IgG fraction). Meantime, with the guinea pig IgE fraction sample, no color developed near a molecular weight of approximately 160 kDa (lane 5; guinea pig IgE fraction). These findings indicated that the guinea pig IgE fraction of the present invention is substantially free from guinea pig IgG.

### Example 2: Production of anti-guinea-pig IgE antibody

After the fraction obtained in Example 1 and an adjuvant (Titer Max: produced by Sigma Ltd.) were mixed in a 1:1 ratio, this mixture was emulsified by sonication and prepared to obtain a ratio of 25 µg of the fraction obtained in Example 1 and 0.5 mL of the emulsion per mouse. This immune antigen was intradermally and percutaneously administered three times at 2-week intervals to mice (BALB/c, 4-week-old). One week after the third immunization, splenocytes were collected from each mouse, and fused with mouse myeloma cells by the PEG method. After the fusion, the hybridoma was dispensed to a 96-well plate and cultured in a 37°C carbon dioxide incubator (5% CO₂, 37°C) for 1 to 2 weeks to select by using HAT medium. An antibody in the supernatant of fused cells was detected by the ELISA method using a guinea pig IgE fraction immobilized microplate, and the presence or absence of the antibody was confirmed. For positive wells, cloning was twice repeated by limiting dilution, and a clone reactive to guinea pig IgE was selected. The monoclonal antibody produced by the clone obtained was purified from ascites fluid from a BALB/c mouse wherein hybridoma cells had been proliferated in the peritoneal cavity of the mouse, using Protein G Sepharose gel.

### Example 3: Production of sandwich ELISA reagent

A reagent for guinea pig IgE assay by the sandwich ELISA method was produced as described below.

### (1) Preparation of horseradish-peroxidase-labeled mouse anti-guinea-pig IgE monoclonal antibody

4 mg of horseradish peroxidase was dissolved in 1 mL of distilled water, 120 µL of 0.1 mol/L sodium periodate solution was added to 600 µL aliquot of the enzyme solution and a reaction was performed at room temperature for 20 minutes. This solution was dialyzed against a 1 mmol/L sodium acetate buffer solution (pH 4.4) overnight to obtain a periodate-oxidized peroxidase solution. 15 µL of a 0.2 mol/L carbonate buffer solution was added to 300 µL of the periodate-oxidized peroxidase solution, the mouse anti-guinea-pig IgE monoclonal antibody produced in Example 2 (2 mg/ml) was mixed thereto, and the mixture was incubated at ambient temperature under shading for 2 hours. Subsequently, 25 µL of 4 mg/mL sodium borohydride was added, and the mixture was allowed to stand at 4°C for 2 hours. The mixture was dialyzed against a 50 mmol/L phosphate buffer solution (pH 7.0) overnight.

### (2) Coating of mouse anti-guinea-pig IgE antibody on solid support

The mouse anti-guinea-pig IgE antibody obtained in Example 2 was dissolved in a buffer solution for antibody liquid (5 mmol/L phosphate, 0.9% NaCl, pH 7.0) to adjust a concentration of 10 µg/mL. This solution was dispensed to a microtiter plate (micromodule plate, Maxsorp-F8, Nunc Company) at 200 µL per well, and allowed to stand at 4°C for 3 nights.

### (3) Blocking of microtiter plates

After each well was washed with 300 µL of plate washing solution (40 mmol/L phosphate, 0.9% NaCl, 0.1% antiseptic [ProClin 150; Rohm and Haas Company], 0.1% bovine serum albumin, pH 7.0) three times, 300 µL of a buffer solution for storage (0.1% ProClin 150, 1% Block Ace [registered trademark] powder; Dainippon Pharmaceutical Co., Ltd.) was added, and the mixture was allowed to stand at room temperature for 2 hours, after which the buffer solution for storage was removed.

### (4) Preparation of standard substance

The guinea pig IgE fraction was subjected to SDS-PAGE under non-reducing conditions in the same manner as described above, and the gel was stained with Coomassie Brilliant Blue. The stained gel was measured using a densitometer (SHIMADZU double-wavelength flying spot scanning densitometer CS-9300PC). The relative staining intensity of the about 200-kDa guinea pig IgE major band was determined to be 79%. This fraction was assayed using the BCA protein assay kit (Pierce Company) to quantify the protein, and the protein concentration was multiplied by 0.79 to obtain the guinea pig IgE concentration in this fraction. This fraction was diluted with fetal bovine serum to prepare 0, 25, 50, 100, 200, 400, and 800 ng/mL of standard solutions.

### Example 4: Measurement of guinea pig IgE using the reagent produced in Example 3

### (1) Generation of standard curve

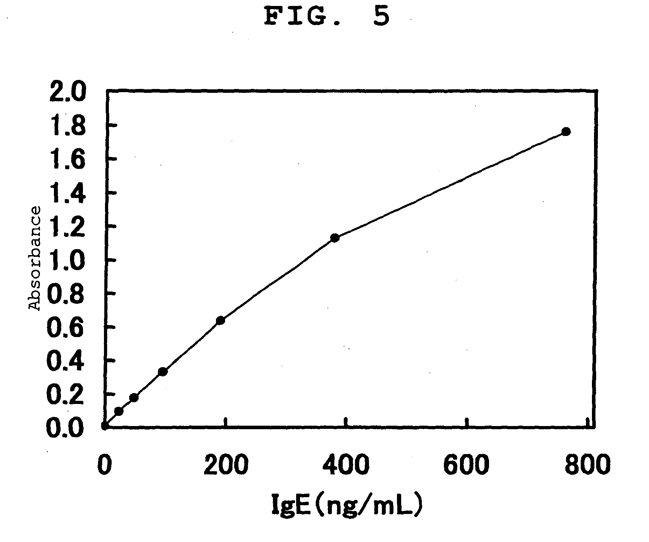

100 µL of a buffer solution for reaction (20 mmol/L 2-morpholinoethanesulfonic acid-NaOH, 0.9% NaCl, 0.1% BSA, 0.2% ProClin 150) was dispensed to each well of a microtiter plate. 25 µL of standard substance (0, 50, 100, 200, 400, and 800 ng/mL) was added to each well in the same manner as described above, and the plate was allowed to stand at room temperature for 1 hour. After each well was washed with 300 µL of a washing solution three times, 100 µl of a dilution of horseradish peroxidase-labeled mouse anti-guinea-pig IgE monoclonal antibody in diluent was added, and the plate was allowed to stand at room temperature for 1 hour. Next, after each well was washed with 300 µL of the washing solution three times, 100 µL of TMB solution was added to each well, and a reaction was performed at room temperature under shading for 30 minutes. Subsequently, 100 µL of 3.2 mol/L sulfuric acid was added to each well to stop the reaction. The absorbance of each well was measured using a microplate reader at a primary wavelength of 450 nm and a secondary wavelength of 630 nm. Figure 5 shows the assay results from serial dilution samples of standard guinea pig IgE (800 ng/ml). This figure indicates that a good standard curve was obtained.

### (2) Basic performance tests of the reagent

### (a) Dilution test

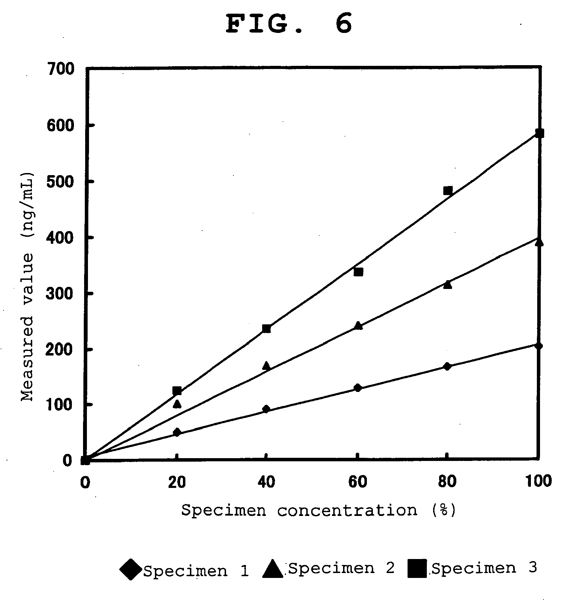

A dilution test of guinea pig serum specimens was performed according to the method of (1) above. Three serum specimens of unknown IgE contents were diluted with a specimen diluent to prepare respective series of dilutions, and the IgE concentrations in the specimens were calculated on the basis of a standard curve generated from simultaneously measured standard guinea pig IgE. As shown in the results of Figure 6, good dilution linearity nearly passing the zero point was obtained.

### (b) Recovery test

A recovery test of guinea pig IgE was performed according to the method of (1) above. Standard guinea pig IgE was added to three serum specimens, and the recovery rate of IgE added was determined from the measured values. As shown in Table 1 below, good results were obtained with the recovery rate being 98.9% to 109.7%.

**[Table 1]**

| Specimen | Added concentration (ng/mL) | Measured concentration (ng/mL) | Theoretical concentration (ng/mL) | Amount recovered (ng/mL) | Recovery rate (%) | Mean recovery rate (%) |
|---|---|---|---|---|---|---|
| A | 0 | 74.7 | 74.7 | - | - | |
| | 178.7 | 249.0 | 253.4 | 174.3 | 97.5 | |
| | 342.5 | 417.9 | 417.2 | 343.2 | 100.2 | 98.9 |
| B | 0 | 82.6 | 82.6 | - | - | |
| | 178.7 | 278.8 | 261.3 | 196.2 | 109.8 | |
| | 342.5 | 458.1 | 425.1 | 375.5 | 109.6 | 109.7 |
| C | 0 | 66.1 | 66.1 | - | - | |
| | 178.7 | 233.2 | 244.8 | 167.1 | 93.5 | |
| | 342.5 | 424.4 | 408.6 | 358.3 | 104.6 | 99.1 |

### (c) Within-run reproducibility test

The same guinea pig serum sample was measured 8 times according to the method of (1) above to perform a within-run reproducibility test. A good result was obtained with the coefficient of variance CV% being not more than 1.8%.

### (d) Cross-reactivity test

Samples of purified guinea pig IgG and purified guinea pig IgM (Cortex Company) were assayed according to the method of (1) above; as a result, the reagent of the present invention hardly cross-reacted with IgG and IgM (the cross-reaction rate was less than 0.0001%), as shown in Table 2 below. Thus, the present assay system was shown to be an assay system that does not cross-react with guinea pig IgG and guinea pig IgM, and to be an assay system specific for guinea pig IgE.

**[Table 2]**

| Guinea pig immunoglobulin | Sample concentration (µg/mL) | Measured value (ng/mL) | Cross-reaction rate (%) |
|---|---|---|---|
| IgE | 0.8 | 800 | 100 |
| IgG | 1429 | 1.2 | 0.00008 |
| IgM | 900 | 0.3 | 0.00003 |

These results of the basic performance tests indicate that the immunoassay reagent of the present invention is capable of measuring guinea pig IgE at very high accuracy.

The reagent for immunoassay of the present invention was found to be capable of detecting 6.2 ng/mL, and even 3.1 ng/mL, of guinea pig IgE present in as small as 25 µL of guinea pig serum, demonstrating a very high sensitivity. Although the reagent for immunoassay of the present invention permits setting a range of measurable guinea pig IgE concentrations as appropriate, a range of about 3 to about 800 ng/mL is preferred in the case of, for example, a blood sample. With the reagent for immunoassay of the present invention, the IgE concentration in blood of normal guinea pigs was determined to be 31.9 to 97.6 ng/mL.

### Example 5: Measurement of OVA-recognizing guinea pig IgE

### (1) Preparation of OVA-recognizing guinea pig IgE standard substance

The guinea pig IgE standard substance prepared in Example 3 was developed on an OVA-immobilized column to adsorb OVA-recognizing guinea pig IgE only. The guinea pig IgE having passed through the column was quantified by the method described in Example 4, and this amount of the IgE was subtracted from the amount of guinea pig IgE developed on the column to calculate the concentration of OVA-recognizing guinea pig IgE adsorbed to the column.

A detailed description is given below.

### (a) Preparation of OVA-immobilized column

NHS-activated Sepharose 4 Fast Flow (produced by Amersham Biosciences K.K.) was taken in a 5-mL column, and isopropanol was removed. After the column was washed with 30 mL of ice cooled 1 mmol/L HCl, 5 mL of a solution of OVA diluted with a coupling buffer solution (0.5 mol/L NaCl, 0.2 mol/L sodium carbohydrate (pH 8.3)) to obtain a final concentration of 10 mg/mL OVA was added, and the column was allowed to stand at room temperature for 30 minutes. The column was washed with 30 mL of a coupling buffer solution, 30 mL of a washing buffer solution (0.5 mol/L NaCl, 0.1 mol/L sodium acetate, pH 4.0), and 30 mL of a blocking buffer solution (0.15 mol/L Tris-HCl, pH 8.8), and the column was allowed to stand at room temperature for 1 hour. The column was washed with 30 mL of the washing buffer solution, 30 mL of the blocking buffer solution, and 30 mL of the washing buffer solution. The OVA-immobilized NHS-activated Sepharose 4 Fast Flow gel was suspended in a developing buffer solution (20 mmol/L sodium phosphate, pH 7.0), and this suspension was packed in another column to obtain a gel volume of 1 mL.

### (b) Measurement of OVA-recognizing guinea pig IgE

concentration by OVA-immobilized column chromatography The OVA-immobilized Sepharose 4 Fast Flow column prepared in the previous term (gel volume: 1 mL) was equilibrated with 10 mL of the same developing buffer solution as the previous term. 100 µL of 760 ng/mL of guinea pig IgE standard substance was developed on the column, and the column was washed with the developing buffer solution. The fraction having passed the column was collected in 1-mL portions. The guinea pig IgE concentration in the collected fraction was quantified by the method described in Example 4. The quantitation results are shown in Table 3 below. The sum of the amounts of guinea pig IgE in the fraction having passed the column was 19.2 ng. The amount of guinea pig IgE having passed the column (19.2 ng) was subtracted from the amount of guinea pig IgE developed on the column (76 ng) to calculate the amount of OVA-recognizing guinea pig IgE adsorbed to the column (56.8 ng). From this finding, it was shown that 568 ng/mL of OVA-recognizing guinea pig IgE was contained in the guinea pig IgE standard substance sample (760 ng/mL) used in the above-described OVA-immobilized column chromatography.

**[Table 3]**

| Fraction No. | Measured value of IgE (ng/mL) | Amount of IgE per fraction (1 ml) (ng) |
|---|---|---|
| 1 | 8.1 | 8.1 |
| 2 | 9.3 | 9.3 |
| 3 | 1.4 | 1.4 |
| 4 | 0.4 | 0.4 |
| 5 | 0 | 0 |
| 6 | 0 | 0 |
| 7 | 0 | 0 |
| | Total | 19.2 |

### (2) Preparation of horseradish peroxidase (HRP)-labeled OVA

### (a) SATA modification of OVA

27 mg of OVA (produced by Sigma Ltd.) was dissolved in 1 mL of a 0.1 mol/L phosphate buffer solution (pH 7.0). A solution diluted with N,N'-dimethylformamide to obtain a final concentration of 24 mmol/L SATA (N-Succinimidyl S-acetylthioacetate, produced by Pierce Company) was prepared, 0.1 mL of this solution was added to the above-described OVA solution, and the mixed solution was incubated at 30°C for 30 minutes, after which 0.1 mL of a 0.1 mol/L EDTA solution (pH 7.0), 0.1 mL of 1 mol/L Tris-HCl (pH 7.0), and 0.15 mL of 1 mol/L hydroxylamine (pH 7.0) were added, and the mixture was incubated at 30°C for 15 minutes. After the mixture was developed over a Hi-Trap G-25 (5 mL) column (developing buffer solution: 0.1 mol/L sodium phosphate, pH 6.0, 5 mmol/L EDTA, flow rate: 0.5 mL/min, fraction volume: 0.5 mL) for purification, an OVA-SATA-modified solution was prepared to obtain a final concentration of 4 mg/mL.

### (b) Preparation of HRP-Sulfo-HMCS

16 mg of HRP (produced by Toyobo Co., Ltd., TYPE I-C) was dissolved in 2.0 mL of a 0.1 mol/L phosphate buffer solution (pH 7.0). 4 mg of Sulfo-HMCS (N-(8-Maleimidocapryloxy)sulfosuccinimide, sodium salt, produced by Dojindo Laboratories Company) was dissolved in 0.275 mL of a 0.1 mol/L phosphate buffer solution (pH 7.0). 0.2 mL of the Sulfo-HMCS solution was added to the entire volume of the above-described HRP solution, and the mixture was incubated at 30°C for 60 minutes. After the mixture was developed on a Hi-Trap G-25 (5 mL) column (developing buffer solution: 0.1 mol/L sodium phosphate, pH 6.0, 5 mmol/L EDTA, flow rate: 0.5 mL/min, fractionated volume: 0.5 mL) for purification, a HRP-Sulfo-HMCS solution was prepared to obtain a final HRP concentration of 7.2 mg/mL.

### (c) HRP labeling of OVA

OVA-SATA-modified solution and an equal volume of the HRP-Sulfo-HMCS solution were mixed, and this mixture was incubated at 4°C for 20 hours. 10 mmol/L cysteamine hydrochloride was added at 20 µL per 1 mL. The mixture was developed on a Sephacryl S-200 XK-26/70 column (developing buffer solution: 0.1 mol/L sodium phosphate pH 6.5, flow rate: 0.8 mL/min, fractionated volume: 2.0 mL), and subjected to gel filtration fractionation. The recovered fraction was determined by measuring the HRP activity and titer of each fraction.

### (3) Generation of standard curve

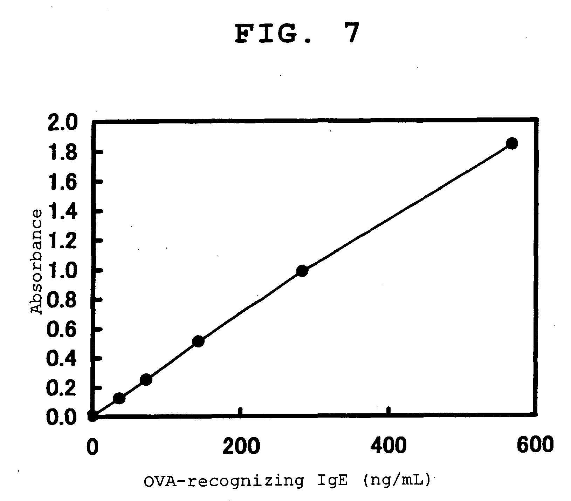

150 µL of a reaction buffer solution (20 mmoL/L 2-morpholinoethanesulfonic acid-NaOH, 0.9% NaCl, 0.1% BSA, 0.2% ProClin 150) was dispensed to each well of a plastic microplate, 15 µL of each of the serial dilutions of standard substance prepared in term (1) above (0, 35.5, 71.0, 141.9, 283.9, and 568 ng/mL) was added to each well in the same manner, and this mixture was stirred, after which the plastic microplate was allowed to stand at room temperature for 10 minutes. A 110-µL sample was taken from each well of the plastic microplate and dispensed to each well of a microtiter plate coated with the mouse anti-guinea-pig IgE antibody prepared in Example 3, and the sample was stirred, after which the microtiter plate was allowed to stand at room temperature for 1 hour. After each well was washed with 300 µL of the washing solution three times, 100 µL of the horseradish-peroxidase-labeled OVA prepared above was added to each well, and the microtiter plate was allowed to stand at room temperature for 30 minutes. Next, each well was washed with 300 µL of the washing solution three times, after which 100 µL of a TMB solution was added to each well, and the reaction was performed at room temperature under shading for 30 minutes. Subsequently, 100 µL of 3.2 mol/L sulfuric acid was added to each well to stop the reaction. The absorbance of each well was measured at a primary wavelength of 450 nm and a secondary wavelength of 630 nm using a microplate reader. The results of measurements of the serial dilution samples of IgE standard substance (568 ng/ml) prepared in term (1) are shown in Figure 7. This figure indicates that a good standard curve was obtained.

### (4) Basic performance tests of reagent

### (a) Dilution test

A dilution test of guinea pig serum specimens was performed according to the method of term (3) above. Three serum specimens of unknown OVA-recognizing IgE contents were diluted with a specimen diluent to prepare respective series of dilutions, and the concentrations of OVA-recognizing IgE in the specimens were calculated on the basis of a standard curve generated in the same manner as term (3) above. As shown in the results of Figure 8, good dilution linearity nearly passing the zero point was obtained.

### (b) Recovery test

A recovery test of OVA-recognizing guinea pig IgE was performed according to the method described in term (3) above. Standard guinea pig IgE prepared in the same manner as term (1) above was added to five serum specimens, and the recovery rate of IgE added was determined from the measured values. As shown in Table 4 below, good results were obtained with the addition-recovery rate being 97.7% to 103.7%.

**[Table 4]**

| Specimen | Added concentration (ng/mL) | Measured concentration (ng/mL) | Theoretical concentration (ng/mL) | Amount recovered (ng/mL) | Recovery rate (%) | Mean recovery rate (%) |
|---|---|---|---|---|---|---|
| A | 0 | 86.7 | 86.7 | - | - | |
| | 183.1 | 278.6 | 269.8 | 191.9 | 104.8 | |
| | 384.3 | 458.6 | 471.0 | 371.9 | 96.8 | 100.8 |
| B | 0 | 164.6 | 164.6 | - | - | |
| | 183.1 | 364.6 | 347.7 | 200.0 | 109.2 | |
| | 384.3 | 541.9 | 548.9 | 377.3 | 98.2 | 103.7 |
| C | 0 | 201.4 | 201.4 | - | - | |
| | 183.1 | 390.9 | 384.5 | 189.5 | 103.5 | |
| | 384.3 | 575.0 | 585.7 | 373.6 | 97.2 | 100.4 |
| D | 0 | 456.4 | 456.4 | - | - | |
| | 183.1 | 642.9 | 639.5 | 186.5 | 101.9 | |
| | 384.3 | 815.9 | 840.7 | 359.5 | 93.5 | 97.7 |
| E | 0 | 0.6 | 0.6 | - | - | |
| | 183.1 | 192.9 | 183.7 | 192.3 | 105.0 | |
| | 384.3 | 380.8 | 384.9 | 380.2 | 98.9 | 102.0 |

### (c) Within-run reproducibility test

The same guinea pig serum sample was measured 8 times according to the method of (3) above to perform a within-run reproducibility test. A good result was obtained with the coefficient of variance CV% being not more than 5%.

### (d) Specificity test

With serum specimens collected from OVA-sensitized guinea pigs (Hartley) and non-sensitized guinea pigs (same line) as the samples, IgE contents were measured using the assay reagents used in Example 4 and this Example, and the values obtained were compared. Sensitization was performed by dissolving 20 mg of OVA (produced by Sigma Ltd.) in 0.8 mL of physiological saline, and intraperitoneally and subcutaneously injecting 0.4 mL of this solution to each guinea pig in the dorsum; 6 weeks after the sensitization, blood was drawn from each guinea pig.

As shown in the measurement results of Table 5 below, IgE was detected in the blood from the non-sensitized guinea pigs when measured using the reagent described in Example 4, and IgE was not detected when measured using the reagent described in this Example. In contrast, in the OVA-sensitized guinea pigs, IgE was detected when measured using any of the two reagents, and the concentration thereof was higher when measured using the reagent described in Example 4. This result means that the reagent described in Example 4 measured OVA-recognizing IgE and other IgE, and that the reagent described in this Example specifically measured OVA-recognizing IgE.

**[Table 5]**

| Guinea pig individual number | Sensitization performed or not | Assay method described in Example 5 (ng/mL) | Assay method described in Example 4 (ng/mL) |
|---|---|---|---|
| 1 | No | 0 | 19.7 |
| 2 | No | 0 | 26.1 |
| 3 | No | 0 | 10.9 |
| 4 | No | 0 | 174.0 |
| 5 | No | 0 | 46.8 |
| 6 | Yes | 174.0 | 438.7 |
| 7 | Yes | 132.2 | 579.6 |
| 8 | Yes | 209.1 | 666.5 |
| 9 | Yes | 79.4 | 428.9 |
| 10 | Yes | 189.1 | 1177.8 |

### (e) Correlation test with PCA reaction

Correlation between the guinea pig PCA reaction, which is an index of OVA-recognizing IgE present in blood and the assay system of this Example was examined as described below. Eighteen Hartley guinea pigs were used, one of which had its blood drawn without OVA sensitization, and serum was obtained according to a conventional method. The remaining 17 animals were sensitized by intraperitoneally and subcutaneously injecting a 25 mg/mL OVA solution in physiological saline in the dorsum. During six weeks following the sensitization, the sensitized guinea pigs were sacrificed over time, blood was drawn, and serum was obtained according to a conventional method. The IgE content in the serum thus obtained was measured by the method described in this Example.

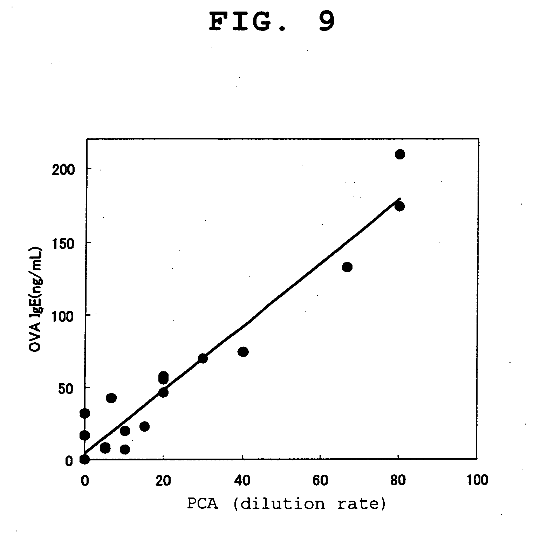

The PCA reaction was performed as described below. The guinea pig serum collected was diluted with physiological saline 10 fold, 20 fold, 40 fold and 80 fold to prepare a series of dilutions. The Hartley guinea pigs were anesthetized with ether and shaven in the back, and 0.1 mL of each serial dilution sample was intradermally injected. After the guinea pigs were reared for 7 days, 1 mL of a solution of 1 mg/mL OVA and 1% Evans Blue in physiological saline was injected from a front leg, and the animals were allowed to stand for 30 minutes. After the guinea pigs were sacrificed, the back skin was removed, and the highest dilution rate at which a blue spot was observed was used as the PCA reaction value. As shown in Figure 9, a very good correlation was observed between the assay system of this Example and the PCA reaction, with the regression equation of y = 2.2x + 4.8 and the correlation factor of r = 0.967. These test results indicate that the immunoassay reagent for guinea pig IgE in this Example is capable of specifically measuring OVA-recognizing IgE.

### Industrial Applicability

The present invention provides a highly purified guinea pig IgE fraction, an excellently specific anti-guinea-pig IgE antibody, and a reagent comprising the antibody, and capable of accurately and conveniently measuring guinea pig IgE. As such, the present invention enables measurement of IgE concentrations in, for example, guinea pig blood, at high sensitivity in animal experiment settings for allergies and the like.

While some of the embodiments of the present invention have been described in detail in the above, it will, however, be evident for those of ordinary skill in the art that various modifications and changes may be made to the particular embodiments shown without substantially departing from the teaching and advantages of the present invention. Such modifications and changes are encompassed in the spirit and scope of the present invention as set forth in the appended claims.

This application is based on patent application Nos. 2003-434618 filed on December 26, 2003 and 2004-289939 filed on October 1, 2004 in Japan, the contents of which are incorporated in full herein by this reference.

## Claims

1. A fraction comprising essentially purified guinea pig immunoglobulin E having a purity sufficient to obtain a single major band of immunoglobulin E by SDS-PAGE, which is separated from the blood of an allergen-sensitized guinea pig, and having the following properties;
(1) having a molecular weight of said major band of about 200 kDa,
(2) being usable as an antigen for producing an antibody capable of specifically recognizing guinea pig immunoglobulin E,
(3) being positive in a passive cutaneous anaphylaxis reaction in guinea pig,
(4) being substantially free of guinea pig immunoglobulin G.

2. The fraction of claim 1, wherein the guinea pig immunoglobulin E has a molecular weight of the major band of 187 to 195 kDa.

3. The fraction of claim 1 or 2, wherein the guinea pig immunoglobulin E has a molecular weight of about 186 kDa as determined by mass spectrometry.

4. The fraction of claims 1 to 3, wherein said major band is separated into two bands by reducing SDS-PAGE, and the molecular weights of the individual bands after the separation are about 70 kDa and about 30 kDa.

5. The fraction of claims 1 to 4, wherein the allergen is ovalbumin.

6. An antibody capable of specifically recognizing guinea pig immunoglobulin E, produced by immunizing a non-human animal with the fraction comprising immunoglobulin E of claims 1 to 5.

7. The antibody of claim 6, wherein the antibody is a monoclonal antibody.

8. The antibody of claim 6 or 7, wherein the antibody exhibits substantially no cross-reaction with guinea pig immunoglobulin G or immunoglobulin M.

9. An antibody capable of specifically recognizing guinea pig immunoglobulin E wherein the cross-reaction rate of the antibody with guinea pig immunoglobulin G or immunoglobulin M is less than 0.0001%.

10. A reagent for immunoassaying guinea pig immunoglobulin E comprising the antibody of claims 6 to 9.

11. A reagent for immunoassaying guinea pig immunoglobulin E comprising the antibody of claims 6 to 9 and an enzyme-labeled antibody thereof.

12. The reagent of claim 10 or 11, wherein the reagent for immunoassay is a reagent for performing sandwich enzyme-linked immunosorbent assay.

13. A reagent for immunoassaying guinea pig immunoglobulin E comprising the immobilized antibody of claims 6 to 9 and ovalbumin labeled with a labeling substance.

14. A reagent for immunoassaying guinea pig immunoglobulin E comprising immobilized ovalbumin and the antibody of claims 6 to 9 as labeled with a labeling substance.

15. The reagent of claim 13 or 14, wherein the labeling substance is an enzyme.

16. The reagent of any of claims 13 to 15, wherein the guinea pig immunoglobulin E is ovalbumin-recognizing immunoglobulin E.

17. The antibody of claims 6 to 9, which is immobilized on a solid phase.

18. The antibody of claim 17, which is used for producing or purifying guinea pig immunoglobulin E.
